(19) Europäisches Patentamt
European Patent Office
Office européen des brevets

(11) **EP 3 484 561 B1**

(12) **EUROPÄISCHE PATENTSCHRIFT**

(45) Veröffentlichungstag und Bekanntmachung des Hinweises auf die Patenterteilung:
**17.03.2021 Patentblatt 2021/11**

(21) Anmeldenummer: **17777770.3**

(22) Anmeldetag: **13.07.2017**

(51) Int Cl.:
*A61M 11/00* *(2006.01)*　　*A61M 15/00* *(2006.01)*

(86) Internationale Anmeldenummer:
**PCT/DE2017/100581**

(87) Internationale Veröffentlichungsnummer:
**WO 2018/010733 (18.01.2018 Gazette 2018/03)**

(54) **INHALATIONSVERFAHREN MIT GESTEUERTER ZYKLISCHER AKTIVIERUNG**

INHALATION METHOD WITH CONTROLLED CYCLIC ACTIVATION

PROCÉDÉ D'INHALATION UTILISANT UNE ACTIVATION CYCLIQUE COMMANDÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.07.2016 DE 102016112986**

(43) Veröffentlichungstag der Anmeldung:
**22.05.2019 Patentblatt 2019/21**

(73) Patentinhaber: **NEBU-TEC med. Produkte Eike Kern GmbH**
**63820 Elsenfeld (DE)**

(72) Erfinder: **KERN, Stefan**
**63820 Elsenfeld (DE)**

(74) Vertreter: **Pöhner, Wilfried Anton**
**Pöhner Scharfenberger & Partner**
**Patent- und Rechtsanwälte mbB**
**Kaiserstrasse 33**
**Postfach 6323**
**97013 Würzburg (DE)**

(56) Entgegenhaltungen:
**DE-A1- 19 720 701　　DE-A1-102006 026 786**
**US-A1- 2010 282 247　　US-A1- 2016 175 545**

**Beschreibung**

Vorliegende Erfindung beschäftigt sich mit einem Vernebler zur Durchführung eines Inhalationsverfahrens gemäß den Merkmalen des Oberbegriffs des Anspruchs 1.

Bei Erkrankungen der Atemwege, insbesondere Kehlkopf, Bronchien, Alveolen oder auch der Lungenbläschen selbst, ist eine Inhalationstherapie häufig die effizienteste und vor allem effektivste Methode, ein Medikament an den gewünschten Einsatzort zu bringen. Zur Durchführung einer solchen Inhalationstherapie werden heutzutage sog. Vernebler eingesetzt, bei welchen eine Medikamentenformulierung, welche in flüssiger Form vorliegt, mittels einer Zerstäubereinheit zu einem feintröpfigen Aerosol zerstäubt wird. Die zu erreichende Tröpfchengröße hängt dabei davon ab, an welcher Stelle der Atemwege die Deponierung des Medikaments erfolgen soll: Bei Erkrankung der Bronchien sind größere Tröpfchendurchmesser zulässig, bzw. nötig als bei Erkrankungen der Lungenbläschen, welche die Tröpfchen des Aersolols nur erreichen können, falls der Tröpfchenradius im Bereich weniger Mikrometer liegt.

Die in modernen Verneblern eingesetzte Zerstäubungseinheit besteht üblicherweise aus einer sog. Mesh-Membran, das ist eine feine Metallmembran mit mikroskopisch kleinen, trichterförmigen Löchern in einem Zentralbereich der üblicherweise einen kreisförmigen Grundriss aufweisenden Membran, und einem an die Membran mechanisch gekoppelten Schwingungserzeuger, der diese in schnelle, typischerweise im Ultraschallbereich liegende Schwingungen versetzt. Die Medikamentenflüssigkeit ist üblicherweise in einem in der Verneblereinheit integrierten Medikamentenreservoir eingeschlossen und steht in direktem Kontakt mit der Zerstäubungseinheit bzw. der Mesh-Membran, sodass beim Schwingen der Membran Medikamentenflüssigkeit durch die trichterförmigen Perforationen der Membran gedrückt wird, und auf der Ausgangsseite der Membran ein Aerosol in Form eines feintröpfigen Nebels erzeugt wird.

Bei den üblichen heutzutage eingesetzten Verneblern besteht der Nachteil, dass die Zerstäubung der Medikamentenflüssigkeit kontinuierlich über die gesamte Dauer des Inhalationsvorgangs hinweg erfolgt, d.h. sowohl während der Patient ein- als auch ausatmet. Beim Ausatemvorgang kann jedoch naheliegenderweise kein Medikament in die Lungen gelangen, wodurch sich das zu dieser Zeit zerstäubte Medikament im Mundstück ausgangsseitig der Zerstäubereinheit niederschlägt und somit ungenutzt verloren geht. Ein Sammeln und Wiederverwenden dieses Niederschlags ist aufgrund hygienischer Bedenken ausgeschlossen, denn der ausgangsseitig des Aerosolerzeugers gelegene Raum ist nicht-steril.

Um diese Verluste zu vermeiden, wäre es also wünschenswert, wenn der Zerstäubungsvorgang nur während des Einatemvorgangs erfolgte. Eine solche gesteuerte Zerstäubung ist dem Stand der Technik für Beatmungsmaschinen bekannt, welche als stationäre, relativ komplexe und teurere Geräte in Krankenhäusern zur Beatmung von Patienten auf der Intensivstation eingesetzt werden. In solchen Maschinen erfolgt eine detaillierte Überwachung des Atemzyklus inklusive Überwachung von Druck und/oder Durchfluss, wodurch eine entsprechende Steuerung eines an eine solche Beatmungsmaschine steuerungstechnisch angeschlossenen Verneblers möglich ist.

Bei mobilen, d.h. tragbaren, Verneblern ist eine Triggerung oder andersartige Steuerung in der Regel nicht bekannt. D.h. solche Geräte zerstäuben während einer Inhalation kontinuierlich die Medikamentenflüssigkeit, mit den oben beschriebenen nachteiligen Konsequenzen. Dies liegt daran, dass die mit einer Überwachung des Atemverlaufes verbundene Sensoreinrichtung das Gerät sehr komplex und damit auch teuer werden lassen würde.

Eine Möglichkeit, eine gesteuerte Aktivierung des Zerstäubungsvorgangs vorzunehmen, welche aufgrund ihrer relativ einfachen Durchführung auch für tragbare Geräte geeignet ist, wird in der Patentschrift EP 1 304 131 B1 beschrieben. Dort wird gelehrt, dass ein Ausgangssignal der Zerstäubungseinrichtung selbst, d.h. des an die Mesh-Membran mechanisch gekoppelten Schwingungserzeugers, welcher üblicherweise ein elektromechanischer Piezokristall ist, dazu verwendet werden kann, den Zerstäubungsvorgang zu starten. Der Nachteil hierbei ist, dass dieses Ausgangssignal von der auf die Membran einwirkende Druckänderung in Folge des beginnenden Atemvorgangs variiert, und somit auch empfindlich auf Erschütterungen des Inhalators reagiert. Dies ist insbesondere bei tragbaren und in der Hand gehaltenen Inhalatoren nachteilig, da diese Erschütterungen unregelmäßig und nicht vorhersagbar auftreten. Auch lässt sich der Verlauf des Atemvorgangs anhand dieses Ausgangssignals nur ungefähr nachvollziehen.

Die Offenlegungsschriften US 2016/0175545 A1 und US 2010/0282247 beschreiben eine Vorrichtung zur Übertragung eines Aerosolnebels von einem Aerosolerzeuger zu einem Patienten und ein Verfahren zur Ansteuerung des Aerosolerzeugers. Die Vorrichtung verfügt über eine Aerosolkammer und einen mit dieser in fluider Kommunikation stehenden oberseitigen Anschluss für eine Druckmessleitung. Der Aerosolerzeuger soll unter anderem abhängig von dem gemessenen Druck so angesteuert werden, dass die Aerosolerzeugung und -abgabe nur zu bestimmten Phasen des Atemzyklus des Patienten erfolgt.

Die Offenlegungsschrift DE 10 2006 026 786 A1 schlägt einen Dosierinhalator vor, welcher dem Patienten anzeigt, wann welche Atemphase stattfinden, dieser beispielsweise mit dem Ausatmen beginnen soll. Eine mit einem Ausatemrohr gekoppelte Ausatemerkennung erkennt, wenn Luft durch das Ausatemrohr strömt.

In der Offenlegungsschrift DE 197 20 701 A1 ist eine Vorrichtung zur Applikation eines Medikament-Aerosols offenbart, welches die Aerosolerzeugung startet, sobald über einen direkt angeschlossenen Drucksensor erfasst wird, dass ein Patient einen Saugdruck im Mundstück erzeugt. Die Aerosolerzeugung wird dann nach einer festen Zeit wieder gestoppt.

Aufgabe vorliegender Erfindung ist es, einen zur Durchführung eines atemabhängig gesteuerten Inhalationsverfahrens geeigneten Vernebler zu entwickeln, welcher eine möglichst effiziente und effektive Medikamentennutzung auch bei tragbarer Ausgestaltung erlaubt.

Diese Aufgabe wird gelöst durch einen Vernebler nach dem unabhängigen Anspruch.

Der Vernebler ist hierbei so ausgestaltet, dass er an der Verneblereinheit, welche als Aerosolerzeuger die Mesh-Membran mit damit gekoppeltem Piezo-Schwingungserzeuger enthält, über einen Luftkanal verfügt, welcher mit einem Ende im Inneren der Verneblereinheit in der ausgangsseitig des Aerosolerzeugers liegenden Aerosolkammer mündet und unterhalb des Aersolerzeugers verlaufend mit seinem zweiten Ende nach hinten geführt ist wo er in einem an die Steuereinheit angeschlossenen, hervorstehenden, kegelförmigen Stutzen endet. Dadurch wird erreicht, dass in der Steuereinheit ein geeigneter Sensor zur Messung eines Drucks und/oder eines Durchflusses durch das Mundstück eingebaut werden kann, der in fluider Kommunikation mit dem Inneren der Verneblereinheit steht.

Vorteil eines solchermaßen ausgestalteten Verneblers ist, dass das erste Ende des Luftkanals an eine möglichst geeignete Stelle im Inneren der Verneblereinheit ausgangsseitig des Aerosolerzeugers münden kann, an welcher eine Druckdifferenz im Laufe des Atemvorgangs maximal ist. Der konkret in der Steuereinheit eingesetzte Sensor zur Überwachung des Drucks und/oder Durchflusses durch das Mundstück ist dann in gewissem Rahmen wählbar. Es kann eine Optimierung hinsichtlich verschiedener Kriterien stattfinden. Zum einen könnte eine technisch möglichst einfache Lösung realisiert werden, bei der ein simpler Drucksensor verwendet wird, welcher auf die Änderung des Gesamtdrucks reagiert. Dieser setzt sich zusammen als Summe des statischem und dynamischen Drucks. Im Laufe eines Atemzuges erniedrigt sich zunächst ersterer, woraufhin sich durch die Aerosolkammer und das Mundstück eine das Aerosol abtransportierende Luftströmung ausbildet, was sich in einem reduzierten dynamischen Druck niederschlägt. Soll nur der eine oder andere Anteil gemessen werden könnte im Inneren der Aerosolkammer eine dem bei Flugzeugen zur Geschwindigkeitsmessung eingesetzten Pitotrohr ähnliche Konstruktion realisiert werden, welche eine automatische Differenzdruckbildung ermöglicht. Für die Zwecke der Aerosolproduktionssteuerung ist aber in der Praxis eine Messung des Gesamtdruckes ausreichend.

Ist eine direkte Strömungs- bzw. Durchflussmessung gewünscht, kann ein beheizter Widerstand eingesetzt und die Temperaturänderung aufgrund des Luftstromes durch den Flusskanal gemessen, oder es kann auch ein miniaturisierter Impeller verwendet werden, der durch den Luftstrom betrieben wird und dessen Drehzahl proportional zur Durchflussmenge ist.

Da die Messgenauigkeit eines solchen dedizierten, externen Sensors höher ist als die eines innerhalb der Verneblereinheit sitzenden, insbesondere auch wenn das Ausgangssignal des Aerosolerzeugers selbst als Sensorsignal und Indikator für den Saugdruck interpretiert wird, ist es möglich, eine besser an den Atemverlauf angepasste Steuerung der Aerosolproduktion zu erreichen.

Das mit dem erfindungsgemäßen Vernebler durchführbare Inhalationsverfahren sieht nun vor, dass der Vernebler, der über einen Druck und/oder Durchflussratensensor verfügt, so angesteuert wird, dass die Zerstäubung/Aerosolproduktion gestartet wird, sobald ein Startkriterium erfüllt ist und gestoppt wird, wenn ein Stoppkriterium erreicht wird.

Die möglichen Startkriterien sind hierbei je nach Empfindlichkeit und Möglichkeiten des Sensors wählbar und schließen ein: Aktivierung bei Überschreiten des Schwellenwerts der Durchflussrate (Luftvolumen pro Zeit), Aktivierung bei Unterschreiten eines Schwellendrucks, Aktivierung bei Erreichen einer bestimmten Gesamtdurchflussmenge (Volumen) oder Aktivierung bei Überschreiten eines Schwellenwertes für eine Zielerreichungswahrscheinlichkeit, welche dynamisch von der Steuereinheit anhand des bisher gemessenen Atemverlaufs errechnet wird. Im einfachsten Fall erfolgt die Aktivierung in Form einer Triggerung, bei der die Inhalation von vollständig aus (0% Aerosolproduktion) auf vollständig an (100% der maximalen Aerosolproduktionsrate) geschaltet wird. Anstelle einer einfachen Triggerung kann auch langsames oder kontinuierliches Erhöhen der abgegebenen Aerosolmenge vorgesehen werden, beispielsweise nach der Formel

$$\frac{\dot{m}}{\dot{m}_{max}} = \text{Min}\left[1, \text{Max}\left[0, \frac{X - X_1}{X_1 - X_2}\right]\right].$$

Hierbei bezeichnet $\dot{m}$ die aktuelle und $\dot{m}_{max}$ die maximale Aerosolproduktionsrate (Masse pro Zeit) und $X$ bzw. $X_1$, $X_2$ den aktuellen Wert bzw. Schwellenwerte der jeweils herangezogenen Steuergröße (Druck, Durchflussrate, Durchflussmenge oder Zielerreichungswahrscheinlichkeit).

Die Stoppkriterien, bei denen die Aerosolerzeugung, d.h. die Zerstäubung beendet wird, können umfassen: Ablauf einer vorgegebenen Zeitspanne, Unterschreiten eines zweiten Schwellenwertes für die Durchflussrate und/oder Überschreiten eines zweiten Schwellenwertes für den Druck und/oder Erreichen eines zweiten Schwellenwertes für die Gesamtdurchflussmenge und/oder Unterschreiten eines zweiten Schwellenwertes für die Wahrscheinlichkeit, dass ein in dem Moment abgegebenes Aerosolpartikel sein Ziel in den Atemwegen des Anwenders erreicht.

Die Schwellenwerte für Aktivierung und Deaktivierung könnten auch identisch gewählt werden, allerdings ist dies in der Praxis meist nicht sinnvoll, da man die Aktivierung für gewöhnlich bei jedem Atemzug so früh wie möglich vornehmen möchte und darum Schwellenwerte wählt, die möglichst nahe am Gleichgewichtswert (ohne Atmung) liegen. Dahingegen sollte das Abschalten/Deaktivieren der Produktion nach Möglichkeit dann erfolgen, wenn das in diesem Moment die Aerosolkammer passierende Luft voraussichtlich nicht mehr ins Zielgebiet gelangen würde, was aufgrund des endlichen Totvolumens in Mundstück, Mundhöhle und ggf. einem Verneblereinheit und Mundstück verbindenden Schlauch, üblicherweise deutlich vor (Wieder)Erreichen des zur Aktivierung verwendeten Schwellenwerts der Fall ist.

Was die Art der Abschaltung betrifft, so ist ebenfalls denkbar, dass neben einem einfachen, sofortigen Ausschalten (Stopp-Triggerung), ein kontinuierliches Absenken der Aerosolproduktion auf Null erfolgt, z.B. indem ähnlich wie oben bei der Aktivierung die Aerosolproduktionsrate proportional zur normierten Differenz einer Steuergröße und einem Schwellenwert gewählt wird. Es können aber auch andere, nicht-proportionale Verläufe vorgesehen werden.

Die Vorteile dieses Verfahrens sind vielfältig. Zum Einen kann in einer einfachen Ausführung eine simple Triggerung erfolgen, welche sehr einfach und zuverlässig funktioniert und zwar sowohl anhand eines erfassten Drucks oder Durchflusswerts. Der wesentliche Vorteil ist, dass eine Aerosolproduktion nicht stattfindet, wenn ein Aerosolteilchen wahrscheinlich nicht sein Ziel erreicht, d.h. insbesondere während des Ausatemvorgangs. Hierdurch stellt das erfindungsgemäße Verfahren zur Durchführung der Inhalation sicher, dass auf einfache Weise und mit einfachen Mitteln, denn es wird nur ein einzelner Sensor benötigt, sichergestellt wird, dass die Aerosolproduktion nur dann erfolgt, wenn sie auch benötigt bzw. wenn das Medikament wirksam werden kann.

Die vorteilhaften Konsequenzen daraus sind zunächst, dass sich ausgangsseitig des Aerosolerzeugers in der Verneblereinheit sowie im Mundstück kein Kondensat aus nicht abgeatmetem und an den Wänden niedergeschlagener Medikamentenflüssigkeit bildet, was den Reinigungsbedarf verringert und insgesamt hygienischer ist. Zum anderen ist die Dichte des Aerosolnebels zu Beginn des Einatmungsvorgangs verringert, was die Tendenz vermindert, dass der Anwender einen Hustenreiz erleidet, der die Inhalation für einige Zeit unterbrechen könnte und durch vorzeitigen Abtransport des Medikaments auch die Wirkung der Inhalation reduzieren oder ganz in Frage stellen kann. Eine Hustenreiztendenz kann vorteilhafterweise weiter verringert werden, wenn bei Erreichen des Aktivierungs- also Startkriterium keine simple Triggerung der Aerosolproduktion erfolgt, sondern die Produktion wie oben beschreiben kontinuierlich auf den Maximalwert hochgefahren wird.

Da die Aerosolproduktion nur erfolgt, wenn sie benötigt wird, wird vorteilhafterweise unter Umständen teures Medikament gespart und damit die Kosten einer Therapie vorteilhafterweise wesentlich verringert. Der Beginn der Aerosolproduktion sowie ihre Beendigung, d. h. der Deaktivierung, ist entweder vor Durchführung der Inhalation oder auch dynamisch während der Inhalation aufgrund der gemessenen tatsächlichen Druck- oder Durchflussverhältnisse möglich. Hierbei können die verwendeten Start- sowie die Stoppkriterien angepasst werden, d.h. die Schwellenwerte für Druck und/oder Durchflussrate oder die bisher ermittelte Gesamtdurchflussmenge oder die Zielerreichungswahrscheinlichkeit werden herauf- oder herabgesetzt. Gegebenenfalls kann auch die Rate geändert werden, in der die Aerosolerzeugung nach einer Triggerung bis auf den Maximalwert erhöht und/oder reduziert wird. Bei einer einfachen sofortigen Beendigung der Aerosolproduktion nach Ablauf einer Zeitspanne, kann diese ebenfalls vorab festgelegt oder dynamisch angepasst werden. Die Steuerung erfolgt in jedem Fall über die Steuereinheit des erfindungsgemäßen Verneblers, bei dem entweder der Benutzer seine gewünschten Parameter manuell einstellt oder die Steuereinheit eine automatische, dynamische Anpassung durchführen lässt.

Ein weiterer, bei tragbaren Verneblern nicht zu vernachlässigender Vorteil besteht darin, dass durch die effizientere, nur zeitweilige Aktivierung der Aerosolproduktion auch die Batterie des Geräts geschont wird.

Weitere vorteilhafte Weiterbildungen vorliegender Erfindung, welche einzeln oder in Kombination realisierbar sind, sofern sie sich nicht gegenseitig offensichtlich ausschließen, sind im Folgenden aufgeführt.

Vorliegende Erfindung schlägt vor, dass im Zuge der Überwachung des Atemverlaufes von der Steuereinheit eine Integration der gemessenen Durchflussrate erfolgt, sodass die gesamt bisher eingeatmete Luftmenge, also das Atemvolumen, ebenfalls bekannt ist. Diese kann dann sowohl zur Aktivierung als auch zur Deaktivierung der Aerosolproduktion herangezogen werden, wobei zum Beispiel eine Aktivierung erfolgt, wenn ein gewisser erster Schwellenwert überschritten wird, und die Deaktivierung erfolgt, wenn ein gewisser zweiter Schwellenwert überschritten wird.

Alternativ kann in der Steuereinheit auch eine ungefähre Zielerreichungswahrscheinlichkeit berechnet werden, welche angibt, wie wahrscheinlich es ist, dass ein zu einer gewissen Zeit produziertes Aerosolpartikel an seinem Zielort in den Atemwegen des Anwenders ankommt. Diese Wahrscheinlichkeit berücksichtigt zum einen den zu erreichenden Zielort, d.h. ob das Aerosol in den Bronchien, den Alveolen oder Lungenbläschen deponiert werden soll, und zum anderen das noch verbleibende Restatemvolumen. Hierbei ist es vorteilhaft, wenn das gesamte Volumen eines Atemzugs bekannt ist. Dieses kann entweder zuvor mit anderen Mitteln gemessenen und der Messwert in Steuereinheit abgelegt werden oder es wird von dem erfindungsgemäßen Vernebler während der Inhalation selbst bestimmt und unter Umständen auch dynamisch an ein sich änderndes Atemverhalten des Anwenders angepasst.

Als Kriterium zum Starten der Aerosolproduktion kommen in Frage: Überschreiten eines Schwellenwertes des Durchflusses, Unterschreiten eines Schwellenwertes des Druckes, Überschreiten einer Gesamtdurchflussmenge, Überschreiten eines Schwellenwertes für die Zielerreichungswahrscheinlichkeit. Als Stoppkriterien kommen in Frage: Ablauf einer Zeitspanne seit Aktivierung der Aerosolproduktion, Unterschreiten eines zweiten Schwellenwertes der Durchflussrate, Überschreiten eines zweiten Schwellenwertes des Druckes, Überschreiten eines zweiten Schwellenwertes der Gesamtdurchflussmenge, und/oder Unterschreiten eines zweiten Schwellenwertes der Zielerreichungswahrscheinlichkeit. Die verwendeten ersten und zweiten Schwellenwerte können hierbei auch identisch sein. Es soll betont werden, dass vorliegende Erfindung vorsieht, dass Start- und Stoppkriterien beliebig miteinander kombinierbar sind, d.h. es kann zum Starten, d.h. Aktivieren der Aerosolproduktion z.B. das Überschreiten eines Schwellenwertes der Durchflussrate, zur Beendigung der Aerosolproduktion aber das Erreichen eines gewissen Schwellenwertes der Gesamtdurchflussmenge eingesetzt werden.

Alle Schwellenwerte können fest vorgegeben und unveränderlich, oder vor der Inhalation vom Anwender selbst wählbar sein. Darüber hinaus ist es denkbar, dass die Steuereinheit eine dynamische Anpassung an tatsächlich gemessene Werte vornimmt. Wird beispielsweise festgestellt, dass eine Gesamtdurchflussmenge, d.h. Atemvolumen, immer weit unter einem Schwellenwert liegt, so kann dieser herabgesetzt werden. Liegt umgekehrt das Atemvolumen deutlich über dem Schwellenwert, so kann dieser heraufgesetzt werden um einen größeren Anteil der Einatemphase auszunutzen.

Schwellenwerte können auch in Kombination angewendet werden, d.h. die Steuereinheit überwacht gleichzeitig mehrere Kriterien und startet oder stoppt die Aerosolproduktion aufgrund des Eintretens aller oder mindestens eines Kriteriums. Dies ist insbesondere bei Verwendung einer Gesamtdurchflussmenge/eines Atemvolumens als Stoppkriterium angebracht. Um zu vermeiden, dass Nichterreichen des Deaktivierungsschwellenwertes, z.B. aufgrund flacher Atmung oder eineres kleinen Lungenvolumens, die Aerosolproduktion einfach weiterläuft, kann zusätzlich die Abschaltung nach Ablauf einer Zeitspanne erfolgen.

Als Verallgemeinerung hierzu schlägt vorliegende Erfindung vor, dass die Steuereinheit für jedes Kriterium einen Erfüllungsgrad, d.h. prozentualer Wert, bestimmt, der, je nach Art der Steuergröße und des Vorgangs (Aktivierung/Deaktivierung), im einfachsten Fall beispielsweise das Verhältnis von Steuergröße zu Schwellenwert, oder invers hierzu Schwellenwert zu Steuergröße ist.

Diese Erfüllungsgrade können dann von der Steuereinheit durch seitens des Anwenders oder auch fest vorgegebener oder auch im Laufe der Inhalation anpassbarer Gewichtungsfaktoren zu einem gewichteten mittleren Erfüllungsgrad verrechnet werden. Aktivierung bzw. Deaktivierung der Aerosolproduktion erfolgt dann bei einem solchen Verfahren bei Erreichen von ersten und zweiten Schwellenwerten des Erfüllungsgrades.

Die Druckmessung erfolgt bevorzugt durch einen Drucksensor in der Steuereinheit, welcher durch den Flusskanal des Verneblers vorliegender Erfindung mit der Ausgangsseite des Aerosolerzeugers in fluider Kommunikation steht, sodass die dortigen Druckänderungen von dem Sensor innerhalb der Steuereinheit registriert werden kann. Alternativ kann auch eine direkte Durchflussmessung erfolgen, entweder indem der Staudruck des Luftstroms durch den Flusskanal gemessen wird oder indem eine Temperaturmessung eines Referenzwiderstands erfolgt oder indem die Drehzahl eines durch den Durchfluss im Flusskanal betriebenen Impellers gemessen wird. In diesem Fall müsste das Steuergerät über einen Lufteinlass verfügen, welcher in fluider Kommunikation mit dem Flusskanal steht.

Weitere Einzelheiten, Merkmale und Vorteile vorliegender Erfindung ergeben sich aus den im Folgenden anhand der Figuren näher erläuterten bevorzugten Ausführungsbeispielen. Diese sollen die Erfindung nur illustrieren und in keiner Weise in ihrer Allgemeinheit einschränken.

Es zeigen im Einzelnen:

Fig. 1:     Längsschnitt durch die Verneblereinheit mit aufgesetztem Mundstück einer bevorzugten Ausführungsform des erfindungsgemäßen Verneblers mit Flusskanal.

Fig. 2:     Beispielgraphen für Durchflussrate und Aerosolproduktion im Verlauf der Zeit bei einer Inhalation gesteuert nach dem mit dem erfindungsgemäßen Vernebler durchführbaren Inhalationsverfahren.

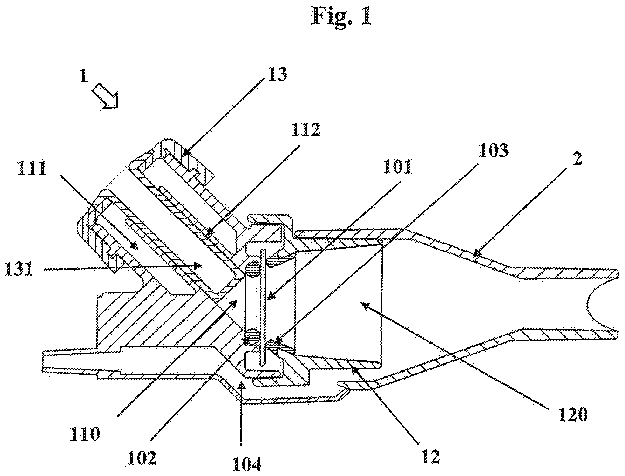

Fig. 1 zeigt einen Längsschnitt durch die Verneblereinheit 1 mit aufgesetztem Mundstück einer Ausführungsform eines Verneblers mit Flusskanal gemäß vorliegender Erfindung. Das Herzstück der Verneblereinheit 1 bildet der Aerosolerzeuger 101, welcher durch Dichtungsring 102 und Haltestruktur 103 mechanisch gehalten ist. Eingangsseitig des Aerosolerzeugers 101 ist das durch eine Kappe 13 fest verschließbare Medikamentenreservoir 11 zu sehen, in welches das flüssige Medikament eingefüllt wird. Ausgangsseitig des Aerosolerzeugers 101 liegt die Aerosolkammer 120, ein im Wesentlichen zylindrisches Volumen, in welchem sich der Medikamentennebel nach der Zerstäubung sammelt, bevor er mit dem durch den Verwender erzeugten Luftstrom durch das Mundstück 2 abtransportiert wird. Das Mundstück 2 ist zur einfacheren Reinigung abnehmbar gestaltet und im Betrieb auf ein an der Verneblereinheit 1 vorhandenes Anschlussstück 12 gesteckt.

An der Unterseite der Verneblereinheit ist der Flusskanal 104 zu sehen, welcher mit seinem vorderem Ende in der ausgangsseitig des Aerosolerzeugers 101 gelegenen Aerosolkammer 120 mündet und mit seinem zweiten Ende nach hinten geführt ist, wo er in einem an die Steuereinheit anschließbaren hervorstehenden kegelstumpfförmigen Stutzen endet.

Die Mündung des Flusskanals 104 bildet den Referenzpunkt der Druckmessung. Sie liegt innerhalb des im Mundstück 2 befindlichen Teils der Aerosolkammer 120, was den entscheidenden Vorteil hat, dass die messbare Gesamtdruckänderung höher ist als bei einem Messpunkt weiter in Richtung Aerosolerzeuger 101. Somit lässt sich einerseits der Atemzyklus des Anwenders genauer überwachen, andererseits ist der dort abgenommene Druck auch kaum von Stößen oder allgemeinen Bewegungen des Verneblers beeinflusst, was die Genauigkeit weiter vorteilhaft erhöht.

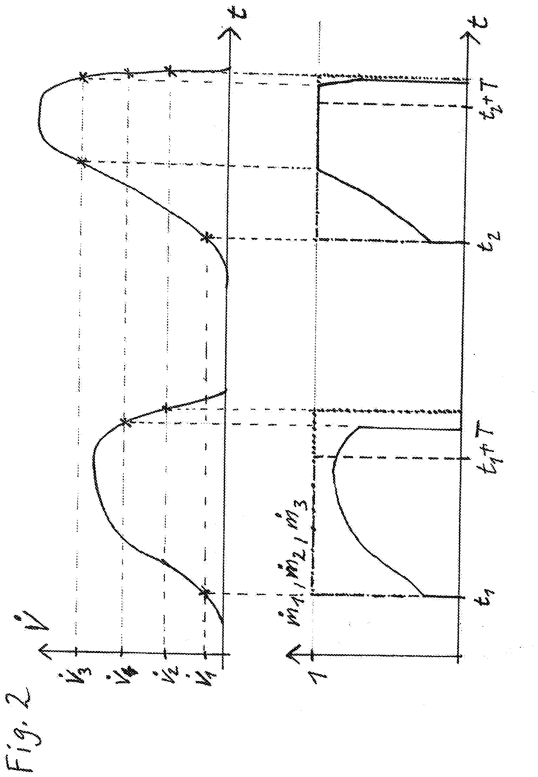

Fig. 2 zeigt zwei Graphen als Beispiel für einen zeitlichen Verlauf der Durchflussrate $\dot{V}$ für zwei Einatmungsvorgänge (oberer Graph) sowie drei verschiedene gemäß vorliegender Erfindung gesteuerter Aerosolproduktionsraten $\dot{m}_1$, $\dot{m}_2$, $\dot{m}_3$ (unterer Graph).

Wie zu sehen ist, wird bei dem ersten Ansteuerungsverfahren, gepunktete Kurve $\dot{m}_1$, die volle Aerosolproduktion getriggert, sobald ein Schwellenwert $\dot{V}_1$ des Flusses überschritten wird, und gestoppt, wenn ein zweiter Schwellenwert $\dot{V}_2$ unterschritten wird.

Der zweite erfindungsgemäße Aerosolproduktionsverlauf, dargestellt als durchgezogene Line $\dot{m}_2$, sieht vor, bei Erreichen des ersten Schwellenwerts $\dot{V}_1$ eine Aktivierung mit verminderter Produktionsrate erfolgt, welche dann bis zu einem Schwellenwert $\dot{V}_3$ hin auf eine maximale Rate erhöht wird, bevor dann bei Unterschreiten dieses Schwellenwertes $\dot{V}_3$ eine Absenkung der Aerosolproduktion beginnt, und diese schließlich bei Erreichen und Unterschreiten des Schwellenwertes $\dot{V}_4$ vollständig deaktiviert wird.

Als Dritte, vielleicht am einfachsten zu realisierende bevorzugte Ansteuerungsart, gestrichelte Kurve $\dot{m}_3$, sieht vorliegende Erfindung vor, nach Triggerung bei Erreichen des Durchflussschwellenwertes $\dot{V}_1$ die Deaktivierung vom Ablauf einer Zeitspanne $T$ abhängig zu machen.

## Bezugszeichenliste

| | |
|---|---|
| 1 | Verneblereinheit |
| 11 | Medikamentenreservoir |
| 12 | Anschlussstück für Mundstück 2 |
| 13 | Kappe |
| 101 | Aerosolerzeuger |
| 102 | Dichtring |
| 103 | Haltestruktur |
| 104 | Flusskanal zum Anschluss an Steuereinheit |
| 2 | Mundstück |
| 21 | Aerosolkammer |
| $\dot{V}$ | Durchflussrate |
| $\dot{V}_1$ | erster Schwellenwert der Durchflussrate |
| $\dot{V}_2$ | zweiter Schwellenwert der Durchflussrate |
| $\dot{V}_3$ | dritter Schwellenwert der Durchflussrate |
| $\dot{V}_4$ | vierter Schwellenwert der Durchflussrate |
| $\dot{m}_1$ | erste Aerosolproduktionsrate |
| $\dot{m}_2$ | zweite Aerosolproduktionsrate |
| $\dot{m}_3$ | dritte Aerosolproduktionsrate |
| $T$ | Zeitspanne |

## Patentansprüche

1. Vernebler zur Durchführung eines Inhalationsverfahrens umfassend die Schritte:

   ◦ Einfüllen von Medikamentenflüssigkeit in ein Reservoir (11) oder Anschließen eines Medikamentenbehälters an ein dafür vorgesehenes Anschlussstück,
   ◦ ggf. Anschließen einer Verneblereinheit (1) an eine Steuereinheit und/oder Aufsetzen eines Mundstücks (2),
   ◦ zumindest zeitweise Aktivierung der Verneblereinheit (1), wobei während der Aktivierung die Medikamentenflüssigkeit zu einem feintröpfigen Aerosol zerstäubt wird, welches in eine durch Verneblereinheit und Mundstück (2) gebildete Aerosolkammer (120) abgegeben wird,
   ◦ Durchführen der Inhalation, wobei ein Anwender das Mundstück mit den Lippen umschließt und beim Einatmen Luft von außen in die Aerosolkammer (120) zieht, wo sich die Luft mit dem Aerosol vermischt, und dann weiter durch das Mundstück (2) in die Atemwege und ggf. die Lunge des Anwenders gelangt,
   wobei durch die Steuereinheit des Verneblers ein Druck in und/oder eine Durchflussrate ($\dot{V}$) durch die Aerosolkammer (120) oder das Mundstück (2) überwacht und die Aerosolproduktion bei jedem Atemzug bei Eintritt eines Aktivierungskriteriums gestartet und bei Erfüllung eines Stopp-Kriteriums wieder gestoppt wird, und wobei der Vernebler umfasst

   - die Verneblereinheit (1) mit dem Reservoir (11),
   - die Steuereinheit mit einem Druck- und/oder Durchflusssensor, an welche die Verneblereinheit (1) anschließbar ist und welche die Ansteuerung eines in der Verneblereinheit (1) eingeschlossenen Aerosolerzeugers (101) steuert,
   - das Mundstück (2) zum Aufsetzen auf ein dafür vorgesehenes Anschlussstück (12) der Verneblereinheit (1), wobei die umfänglich durch Anschlussstück (12) und Mundstück (2) sowie Stirnseitig durch den Aerosolerzeuger (101) begrenzte Aerosolkammer (120) gebildet ist,

**dadurch gekennzeichnet, dass**
- an der Verneblereinheit (1) ein Luftkanal (104) vorhanden ist, der mit seinem ersten Ende ausgangsseitig des Aerosolerzeugers (101) in der Aerosolkammer (120) mündet, und unterhalb des Aerosolerzeugers 101 verlaufend mit seinem zweiten Ende nach hinten geführt ist, wo er in einem an die Steuereinheit angeschlossenen hervorstehenden kegelförmigen Stutzen endet.

2. Vernebler nach Anspruch 1, **dadurch gekennzeichnet, dass** er dazu vorbereitet ist, als Startkriterium das Überschreiten eines Schwellenwerts für den Druck und/oder die Durchflussrate ($\dot{V}$) zu verwenden.

3. Vernebler nach Anspruch 2, **dadurch gekennzeichnet, dass** er dazu vorbereitet ist, aus einem Erfüllungsgrad der Aktivierungskriterien ein gewichtetes Mittel zu bilden und die Inhalation zu starten, sobald dieser Mittelwert einen vorgegebenen Schwellenwert überschreitet.

4. Vernebler nach Anspruch 1 oder 2, **dadurch gekennzeichnet, dass** er dazu vorbereitet ist, eine Zertäubungsrate ($\dot{m}_2$) abhängig von der Differenz zwischen aktuellem Druck und/oder aktueller Durchflussrate ($\dot{V}$) und eines Schwellenwertes für Druck und/oder Durchflussrate ($\dot{V}$) zu steuern.

5. Vernebler nach einem der vorhergehenden Ansprüche, **dadurch gekennzeichnet, dass** die Steuereinheit des Verneblers eine Gesamtdurchflussmenge durch zeitliches Aufintegrieren der gemessenen Durchflussrate ($\dot{V}$) errechnet.

6. Vernebler nach einem der vorhergehenden Ansprüche, **dadurch gekennzeichnet, dass** die Steuereinheit des Verneblers mehrfach während eines jeden Atemzugs eine Zielerreichungswahrscheinlichkeit berechnet.

7. Vernebler nach einem der vorhergehenden Ansprüche, **dadurch gekennzeichnet, dass** er dazu vorbereitet ist, als Stoppkriterium eines oder mehrere der folgenden Ereignisse

- Ablauf einer Zeitspanne ($T$),
- Überschreiten eines zweiten Schwellendrucks,
- Unterschreiten eines zweiten Schwellenflusses ($\dot{V}_2$),

- Erreichen einer vorgegebenen Gesamtdurchflussmenge, und/
oder
- Unterschreiten einer vorberechneten Zielerreichungswahrscheinlichkeit
zu verwenden.

8. Vernebler nach dem vorhergehenden Anspruch, **dadurch gekennzeichnet, dass** er dazu vorbereitet ist, aus einem Erfüllungsgrad der Stoppkriterien ein gewichtetes Mittel zu bilden und die Aerosolproduktion zu stoppen, sobald dieser Mittelwert einen vorgegebenen Schwellenwert überschreitet.

9. Vernebler nach Anspruch 3 oder 8, **dadurch gekennzeichnet, dass** er dazu vorbereitet ist, bei der Mittelung eingesetzte Gewichtungsfaktoren im Laufe der Inhalation anzupassen.

10. Vernebler nach Anspruch 8 und 9, **dadurch gekennzeichnet, dass** er dazu vorbereitet ist, zu Beginn der Inhalation vorgegebene Stoppkriterien, insbesondere der Ablauf einer Zeitspanne ($T$), stärker zu gewichten und diese Gewichtung im Laufe der Inhalation zugunsten anwenderangepasster Stoppkriterien, insbesondere Erreichen einer Gesamtdurchflussmenge oder Unterschreiten einer Zielerreichungswahrscheinlichkeit, zu verändern.

11. Vernebler nach einem der vorhergehenden Ansprüche, **dadurch gekennzeichnet, dass** eine Durchflussmessung mittels

- Messung des dynamischen Drucks im Mundstück (2) oder eines zum Mundstück (2) führenden Luftkanals (104),
- Temperaturmessung eines dem Luftstrom ausgesetzten Referenzwiderstandes, und/oder
- Drehzahlmessung eines mit dem Luftstrom getriebenen Impellers erfolgt.

12. Vernebler nach einem der vorhergehenden Ansprüche, **dadurch gekennzeichnet, dass** er dazu vorbereitet ist, ein zum Stoppen der Aerosolproduktion verwendeten Schwellenwert der Gesamtdurchflussmenge im Laufe der Inhalation an tatsächlich vorkommende, dem Atemvolumen entsprechende, Gesamtdurchflussmengenwerte anzupassen.

**Claims**

1. Nebulizer for carrying out an inhalation procedure comprising the steps:

◦ filling medicament liquid into a reservoir (11) or connecting a medicament container to a connecting piece provided therefor,

○ if applicable, connecting a nebulizer unit (1) with a control unit and/or attaching a mouthpiece (2),

○ at least temporary activation of the nebulizer unit (1), whereby during the activation the medicament liquid is atomized into a fine aerosol, which is dispensed into an aerosol chamber (120) formed by the nebulizer unit and the mouthpiece (2),

○ carrying out the inhalation, whereby a user encloses the mouthpiece with the lips and, when inhaling, draws air from the outside into the aerosole chamber (120), where the air mixes with the aerosol and is further transported through the mouthpiece (2) into the respiratory tracts and, if applicable, the lungs of the user, wherein, by the control unit, a pressure in or a flow rate ($\dot{V}$) through the aerosol chamber (120) is monitored and, during each breathing cycle, the aerosol production is started upon the occurance of an activation criterion and stopped again on the satisfaction of a stopping criterion, and whereby the nebulizer comprises

- the nebulizer unit (1) with the reservoir (11),
- the control unit with a pressure and/or flow rate sensor, to which the nebulizer unit (1) is connectable and which controls the activation of an aerosol generator enclosed in the nebulizer unit (1),
- the mouthpiece (2) for attaching to a connection piece (12) of the nebulizer unit (1) provided therefor, whereby the aerosol chamber (120) is formed, the aerosol chamber being limited circumferentially by the connection piece (12) and the mouthpiece (2) and frontally by the aerosol generator (101),

**characterized in that**

- on the nebulizer unit (1) an air channel (104) is present, which with its first end leads into the aerosol chamber (120) on the output side of the aerosol generator (101), and, running below the aerosol generator (101), with its other end is lead rearwards, where it ends in a protruding cone-shaped stub connected to the control unit.

2. Nebulizer according to claim 1, **characterized in that** it is configured to use as starting criterion the exceeding of a threshold value for the pressure and/or the flow rate ($\dot{V}$).

3. Nebulizer according to claim 2, **characterized in that** it is configured to calculate a weighted average of a degree of fulfilment of the activation criteria and to start the inhalation as soon as this average exceeds a predetermined threshold.

4. Nebulizer according to one of the preceding claims, **characterized in that** it is configured to control a rate of atomization ($\dot{m}_2$) dependent on the difference between a current pressure and/or a current flow rate ($\dot{V}$) and a threshold value of the pressure and/or the flow rate ($\dot{V}$).

5. Nebulizer according to one of the preceding claims, **characterized in that** the control unit of the nebulizer computes a total throughput by temporal integration of the measured flow rate ($\dot{V}$).

6. Nebulizer according to one of the preceding claims, **characterized in that** the control unit of the nebulizer computes, multiple times during each breathing cycle, a probability of reaching a target destination.

7. Nebulizer according to one of the preceding claims, **characterized in that** it is configured to use as stopping criterion one or more of the following events

- the expiry of a time period ($T$),
- the exceeding of a second threshold pressure,
- the falling below a second threshold flow rate ($\dot{V}_2$),
- the reaching of a predetermined total throughput, and/or
- the falling below a pre-computed probability of reaching a target destination.

8. Nebulizer according to the preceding claim, **characterized in that** it is configured to calculate a weighted average of a degree of fulfilment of the stopping criteria and to stop the aerosol production as soon as this average exceeds a predetermined threshold.

9. Nebulizer according to claim 3 or 8, **characterized in that** it is configured to adjust the weighing factors used in the averaging in the course of the inhalation.

10. Nebulizer according to claim 8 and 9, **characterized in that** it is configured to weight predetermined stopping criteria, in particular the expiry of a time period ($T$), more highly at the beginning of an inhalation and to change this weighting in the course of the inhalation in favour of user-adapted stopping criteria, in particular the reaching of a total throughput or falling below a probability of reaching a target destination.

11. Nebulizer according to one of the preceding claims, **characterized in that** a throughput is determined by means of

- measurement of the dynamic pressure in the mouthpiece (2) or in an air channel (104) leading into the mouthpiece (2),

- temperature measurement of a reference resistor exposed to the airstream, and/or
- revolution speed measurement of an impeller driven by the airstream.

12. Nebulizer according to one of the preceding claims, **characterized in that** it is configured to adapt a threshold value of the total throughput used in stopping the aerosol production to actually occuring total throughput values corresponding to the respiratory volume.

**Revendications**

1. Nébuliseur destiné à exécuter un procédé d'inhalation comprenant les étapes :

  ◦ Remplissage de fluide médicamenteux dans un réservoir (11) ou raccordement d'un récipient de médicaments à un raccord prévu à cet effet,
  ◦ le cas échéant raccordement d'une unité de nébuliseur (1) à une unité de commande et/ou pose d'un embout buccal (2),
  ◦ au moins temporairement activation de l'unité de nébuliseur (1), sachant que, pendant l'activation, le fluide médicamenteux est pulvérisé en un aérosol de fines gouttes, qui est remis dans une chambre d'aérosol (120) constituée de l'unité de nébuliseur et de l'embout buccal (2),
  ◦ Réalisation de l'inhalation, dans laquelle un utilisateur entoure l'embout buccal avec les lèvres et, lors de l'inhalation, aspire de l'air de l'extérieur dans la chambre d'aérosol (120), où l'air se mélange à l'aérosol et parvient ensuite à travers l'embout buccal (2) dans les voies respiratoires et le cas échéant le poumon de l'utilisateur,
  dans laquelle l'unité de commande du nébuliseur surveille une pression dans et/ou un débit ($\dot{V}$) à travers la chambre d'aérosol (120) ou l'embout buccal (2) et démarre la production d'aérosol à chaque inspiration lorsqu'un critère d'activation est rempli et l'arrête à nouveau lorsqu'un critère d'arrêt est rempli, et
  dans laquelle le nébuliseur comprend

    - l'unité de nébuliseur (1) avec le réservoir (11),
    - l'unité de commande pourvue d'un capteur de pression et/ou de débit, auquel l'unité de nébuliseur (1) peut être raccordée et laquelle pilote un générateur d'aérosol (101) incorporé dans l'unité de nébuliseur (1),
    - l'embout buccal (2) à poser est formé sur un raccord (12) de l'unité de nébuliseur (1) prévu à cet effet, dans laquelle la chambre d'aérosol (120) est limitée dans sa circonférence par le raccord (12) et l'embout buccal (2), et à l'avant par le générateur d'aérosol (101),
    **caractérisée en ce que**
    - sur l'unité de nébuliseur (1) se trouve un conduit d'air (104), qui débouche avec sa première extrémité du côté de la sortie du générateur d'aérosol (101) dans la chambre d'aérosol (120), et, se dérouler sous le générateur d'aérosol (101) est guidé avec sa deuxième extrémité vers l'arrière, ou il se termine par une buse conique en saillie et raccordé à l'unité de commande.

2. Nébuliseur selon la revendication 1, **caractérisé en ce qu'**il est préparé pour utiliser en tant que critère de démarrage le dépassement d'une valeur de seuil pour la pression et/ou le débit ($\dot{V}$).

3. Nébuliseur selon la revendication 2, **caractérisé en ce qu'il** est préparé pour former une moyenne pondérée à partir d'un degré d'atteinte des critères d'activation et de démarrer l'inhalation dès que cette valeur moyenne dépasse une valeur de seuil prescrite.

4. Nébuliseur selon la revendication 1 ou 2, **caractérisé en ce qu'il** est préparé pour piloter une vitesse de pulvérisation ($\dot{m}_2$) en fonction de la différence entre la pression actuelle et/ou le débit actuel ($\dot{V}$) et d'une valeur de seuil pour la pression et/ou débit ($\dot{V}$).

5. Nébuliseur selon l'une quelconque des revendications précédentes, **caractérisé en ce que** l'unité de commande du nébuliseur calcule un débit total par intégration temporelle du débit mesuré ($\dot{V}$).

6. Nébuliseur selon l'une quelconque des revendications précédentes, **caractérisé en ce que** l'unité de commande du nébuliseur calcule plusieurs fois pendant chaque inhalation une vraisemblabilité d'atteinte de l'objectif.

7. Nébuliseur selon l'une quelconque des revendications précédentes, **caractérisé en ce qu'**il est préparé pour utiliser, en tant que critère d'arrêt, un ou plusieurs des événements suivants

    - terme d'un laps de temps ($T$),
    - dépassement d'une seconde pression de seuil,
    - passage en dessous d'un second flux seuil ($\dot{V}_2$),
    - atteint d'un débit total prescrit, et/ou
    - passage en dessous d'une vraisemblabilité d'atteinte de l'objectif calculée à l'avance.

8. Nébuliseur selon la revendication précédente, **caractérisé en ce qu'il** est préparé pour former une moyenne pondérée à partir d'un degré d'atteinte des critères d'arrêt, et arrêter la production d'aérosol dès que cette valeur moyenne dépasse une valeur de seuil prescrite.

9. Nébuliseur selon la revendication 3 ou 8, **caractérisé en ce qu'**il est préparé pour adapter les facteurs de pondération utilisés lors du calcul de la moyenne au cours de l'inhalation.

10. Nébuliseur selon la revendication 8 et 9, **caractérisé en ce qu'**il est préparé pour, au début de l'inhalation, augmenter la pondération des critères d'arrêt prescrits, notamment du terme d'un laps de temps (T), et de modifier cette pondération au cours de l'inhalation en faveur de critères d'arrêt adaptés à l'utilisateur, notamment l'atteinte d'un débit total ou le passage en dessous d'une vraisemblabilité d'atteinte de l'objectif.

11. Nébuliseur selon l'une quelconque des revendication précédentes, **caractérisé en ce qu'**une mesure de débit est réalisée au moyen

    - de la mesure de la pression dynamique dans l'embout buccal (2) ou d'un conduit d'air (104) menant à l'embout buccal (2),
    - de la mesure de la température d'une résistance de référence exposée au flux d'air, et/ou 6
    - de la mesure de la vitesse de rotation d'une turbine entraînée par le flux d'air.

12. Nébuliseur selon l'une quelconque des revendication précédentes, **caractérisé en ce qu'**il est préparé pour adapter une valeur de seuil du débit total utilisée pour arrêter la production d'aérosol au cours de l'inhalation à des valeurs de débit total correspondant au volume respiratoire apparaissant effectivement.

**Fig. 1**

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1304131 B1
- US 20160175545 A1
- US 20100282247 A
- DE 102006026786 A1
- DE 19720701 A1